(19) Europäisches Patentamt
European Patent Office
Office européen des brevets

(11) Veröffentlichungsnummer : **0 373 385 B1**

(12) # EUROPÄISCHE PATENTSCHRIFT

(45) Veröffentlichungstag der Patentschrift :
**13.01.93 Patentblatt 93/02**

(51) Int. Cl.$^5$ : **C07C 271/16,** C07C 271/18, C08F 14/18, A61K 6/083

(21) Anmeldenummer : **89121424.9**

(22) Anmeldetag : **20.11.89**

(54) **Verwendung von Perfluoralkylgruppen aufweisenden (Meth-)acrylsäureestern in der Dentaltechnik.**

(30) Priorität : **10.12.88 DE 3841617**
**10.12.88 DE 3844619**

(43) Veröffentlichungstag der Anmeldung :
**20.06.90 Patentblatt 90/25**

(45) Bekanntmachung des Hinweises auf die Patenterteilung :
**13.01.93 Patentblatt 93/02**

(84) Benannte Vertragsstaaten :
**AT BE CH DE ES FR GB GR IT LI LU NL SE**

(56) Entgegenhaltungen :
**EP-A- 0 193 370**
**US-A- 4 616 073**

(73) Patentinhaber : **Th. Goldschmidt AG**
**Goldschmidtstrasse 100 Postfach 101461**
**W-4300 Essen 1 (DE)**
Patentinhaber : **GDF GESELLSCHAFT FÜR DENTALE FORSCHUNG UND INNOVATIONEN GMBH**
**Dieselstrasse 6**
**W-6365 Rosbach (DE)**

(72) Erfinder : **Fock, Jürgen, Dr.**
**Mörsenbroicher Weg 114**
**W-4000 Düsseldorf 30 (DE)**
Erfinder : **Hahn, Günter, Dr.**
**Eigene Scholle 35**
**W-4330 Mülheim/Ruhr (DE)**
Erfinder : **Wagenknecht, Günther**
**Bahnhofstrasse 7a**
**W-6363 Echzell (DE)**

EP 0 373 385 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft die Verwendung von Perfluoralkylgruppen aufweisenden (Meth-)acrylsäureestern in der Dentaltechnik, insbesondere von solchen Verbindungen, die als härtbare Makromonomere im Dentalbereich zur Unterfütterung von Zahnprothesen geeignet sind.

Der Ausdruck (Meth-)acrylsäureester soll dabei bedeuten, daß sowohl Methacrylsäureester wie Acrylsäureester gleichermaßen von der Erfindung erfaßt werden.

Aus der Literatur sind fluorhaltige monomere und oligomere (Meth-)acrylate bekannt. Sie werden zur Herstellung dentaler Prothesen- und Füllungsmaterialien verwendet und verleihen diesen verminderte Wasseraufnahme und verringerte Löslichkeit.

Beispielsweise ist im J.Dent.Res., 58, 1981 bis 1986, die Verwendung von 1,1,5-Trihydro-octafluoropentyl-methacrylat als polymerisierbarer Bestandteil in Zahnfüllmassen beschrieben. Außerdem sind fluorhaltige Phenylcarbinol-acrylate, wie 1,1,1,3,3,3-Hexafluor-2-phenyl-2-acryloyloxy-propan aus Org.Coat-Plast-Chem. 42, 204 bis 207, 1980, bekannt.

Weiter werden in der US-PS 4 356 296 ähnliche Verbindungen und ihre Verwendung auf dem Dentalgebiet beschrieben. Die US-PS 4 616 072 offenbart Perfluoralkylmonomethacrylate als hydrophobe Copolymerisate für dentale Füllungsmaterialien. Ebenfalls für die restaurative Zahnmedizin dienen die in den EP-A2-0 201 031 und 0 201 778 offenbarten Monomeren mit substituiertem Bis-phenyltetrafluorethan.

Diese vorbekannten Monomere weisen den Nachteil auf, daß bei ihrer Aushärtung im wesentlichen hartspröde Polymerisate entstehen, was ihre Verwendungsmöglichkeit in der Dentaltechnik stark einschränkt.

Bekannt sind auch Perfluoralkylalkoxyalkyl(meth-)acrylate und deren Polymere. In den US-PS 3 660 360 und 4 080 507 werden diese Verbindungen als wasser- und ölabweisende Mittel für verschiedene Substrate beschrieben, Anwendungen im Dentalsektor sind nicht bekannt.

Der Erfindung liegt die Aufgabe zugrunde, härtbare Makromonomere für den Einsatz in der Dentaltechnik bereitzustellen, welche eine reduzierte Löslichkeit und eine erhöhte mechanische Festigkeit des ausgehärteten Endproduktes aufweisen und insbesondere als Unterfüllungsmaterial für Dentalprothesen mit einer erhöhten Haftung zu bereits ausgehärtetem Polymethylmethacrylat verwendet werden können.

Überraschenderweise konnte diese Aufgabe durch die Verwendung von Gemischen von (Meth-)acrylsäureestern der allgemeinen Formel

$$R^1\text{-}(C_nF_{2n}\text{-})\text{-}(CH_2\text{-})_a\text{-}O\text{-}(C_mH_{2m}O\text{-})_b\text{-}\underset{\underset{O}{\|}}{C}\text{-}\underset{\underset{R^2}{|}}{C}{=}CH_2$$

wobei $R^1$ gleich oder verschieden und ein Wasserstoff- oder Fluorrest ist,

$R^2$ gleich oder verschieden und ein Wasserstoff- oder Methylrest ist,

a einen Wert von 0, 1, 2, 3 oder 4,

b einen durchschnittlichen Wert von 2 bis 30,

n einen durchschnittlichen Wert von 4 bis 12,

m einen durchschnittlichen Wert von 3 bis 14

hat, Makromonomere als härtbare mit der Maßgabe, daß im Molekülgemisch keine Oxyethylengruppen enthalten sind, im Dentalbereich gelöst werden.

Solche Verbindungen können nach an sich bekannten Verfahren, wie beispielsweise in den US-PS 3 660 360 und 4 080 507 beschrieben, hergestellt werden.

Die vorstehende Formel ist als durchschnittliche, allgemeine Formel eines Makromonomeren gemisches zu verstehen. Dabei unterscheiden sich die einzelnen Individuen insbesondere durch die Anzahl ihrer Oxyalkylengruppen, die mit dem durchschnittlichen Wert von b als Maximum einer Schulz-Flory-Verteilung entspricht oder dieser angenähert ist.

Die Kettenlänge der Fluoralkylgruppe wird durch den Index n bestimmt. n hat dabei einen durchschnittlichen Wert von 4 bis 12. Ist der zugrundeliegende Alkohol $R^1C_nF_{2n}\text{-}(CH_2\text{-})_aOH$ eine einzelne Verbindung, ist der Wert von n absolut und entspricht einer ganzen Zahl von 4 bis 12. Bevorzugt für die erfindungsgemäße Verwendung sind Verbindungen mit einem durchschnittlichen oder absoluten Wert von n = 6 bis 10.

Die Hydroxylfunktion des Fluoralkohols kann durch eine oder mehrere $CH_2$-Gruppe(n) von der Perfluoralkylgruppe getrennt sein. Die Anzahl solcher $CH_2$-Gruppen ergibt sich aus dem Wert von a. a kann eine ganze Zahl, nämlich 0, 1, 2, 3 oder 4 sein.

Der Index m der Oxyalkylengruppe $C_mH_{2m}O$- hat einen durchschnittlichen Wert von 3 bis 14. Die

Oxyalkylengruppe kann dabei die Struktur

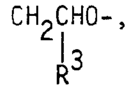

wobei $R^3$ ein Alkylrest mit 1 bis 2 Kohlenstoffatomen ist, oder $CH_2CH_2CH_2CH_2O$- haben, wenn für die Herstellung des fluorierten Alkanolpolyethers Tetrahydrofuran verwendet wird.

Beispiele von geeigneten Oxyalkylengruppen sind der Oxypropylen-,Oxybutylen-, Oxyoctylen-, Oxydecylen- und Oxydodecylenrest. Die Verbindungen für die erfindungsgemäße Verwendung können innerhalb des individuellen Moleküls gleiche oder verschiedene Oxyalkylengruppen aufweisen, so daß der Index m in dem einen Fall als absoluter, in dem anderen Fall als durchschnittlicher Zahlenwert zu verstehen ist. Liegen im gleichen Molekül verschiedene Oxyalkylengruppen nebeneinander - statistisch oder blockweise angeordnet - vor, soll das Molekül frei von Oxyethylengruppen sein. Weist das Molekül nur gleiche Oxyalkylengruppen auf, ist also der Wert m als absolut zu verstehen, folgt aus der Untergrenze von m = 3, daß Oxyethylengruppen ausgeschlossen sind.

Verbindungen, die Perfluoralkylgruppen enthalten, sind in der Regel in üblichen Lösungsmitteln schwer löslich oder unlöslich und deshalb schwierig zu handhaben. Durch die Anwesenheit von Oxyalkylengruppen mit längerkettigen Resten $R^3$ können die Eigenschaften der Verbindungen, insbesondere nach der Polymerisation, wie Hydrophobie und elastisch/plastisches Verhalten, sowie ihre Löslichkeit beeinflußt und dem erfindungsgemäßen Anwendungszweck angepaßt werden.

Die Anzahl der Oxyalkylengruppen ergibt sich aus dem Wert von b und beträgt im Mittel 2 bis 30. Bevorzugt ist ein b-Wert von 5 bis 20.

Besonders bevorzugt für die erfindungsgemäße Verwendung sind solche Verbindungen, bei denen im durchschnittlichen Molekül mindestens 50 Mol-% der Oxyalkylen-Einheiten Oxypropylen- und/oder Oxybutylen-Einheiten sind und der durchschnittliche Wert von m bei den übrigen Oxyalkylen-Einheiten 5 bis 14 beträgt. Es hat sich gezeigt, daß diese Verbindungen für den erfindungsgemäßen Verwendungszweck besonders geeignet sind.

Insbesondere bevorzugt für die erfindungsgemäße Verwendung sind Verbindungen, die im durchschnittlichen Molekül mindestens 90 Mol-% der Oxyalkylen-Einheiten Oxypropylen- und/oder Oxybutylen-Einheiten sind und der durchschnittliche Wert von m bei den übrigen Oxyalkylen-Einheiten 5 bis 14 beträgt.

Verbindungen, bei denen die Oxyalkylen-Einheiten ausschließlich aus Oxypropylen- und/oder Oxybutylen-Einheiten bestehen, verbinden hervorragende Eigenschaften mit niedrigem Kosteneinsatz.

Bei der erfindungsgemäßen Verwendung der Verbindungen als härtbare Makromonomere im Dentalbereich werden diese mit in der Dentaltechnik üblichen Zusatzstoffen compoundiert. Solche Zusatzstoffe können Füllmittel, wie - insbesondere hydrophobierte - Glaskeramik, feinteilige Kieselsäure oder Pigmente oder Modifizierungsmittel sein. Die Modifizierungsmittel dienen dazu, bestimmte anwendungstechnisch wichtige Eigenschaften, wie Elastizität, Reißfestigkeit, Alterungsbeständigkeit, Verträglichkeit, zu optimieren.

Weitere geeignete Modifizierungsmittel sind Divinylbenzol, Ethylenglykoldimethacrylat, Butandioldimethacrylat, Trimethylolpropantrimethacrylat und Pentaerythrittetramethacrylat.

Den Zubereitungen werden ferner Katalysatoren für die strahlungsinduzierte Polymerisation, wie Benzildimethylketal, 2,3-Bornandion, Dimethylaminobenzolsulfansäure-bis-allylamid, Benzophenon, Diethoxyacestophenon, in Mengen von 0,1 bis 3 Gew.-% zugesetzt. Die Härtung der Zubereitung erfolgt mit Hilfe in der Dentaltechnik üblicher Lampen, deren Strahlung eine Wellenlänge von 200 bis 550 nm hat.

Die Härtung kann auch mit peroxidischen Katalysatoren oder Initiatoren bei erhöhten Temperaturen durchgeführt werden. Hierzu werden Peroxide, wie Dibenzoylperoxid, eingesetzt. Bei niedrigen Temperaturen ist eine Vernetzung mit Hilfe von Redox-Inditiatoren möglich. Beispiel eines solchen Redox-Initiators ist das System Dibenzoylperoxid/N,N-Dihydroxyethyl-p-toluidin.

Die Compoundierung mit diversen Additiven und die Aushärtung der (Meth-)acrylsäureester gemische zu anwendungstechnisch optimalen Dentalprodukten entsprechend der erfindungsgemäßen Verwendung geschieht in an sich bekannter Weise und kann beispielsweise den oben zitierten Publikationen, insbesondere den EP-A2-0 201 031 und 0 201 778, entnommen werden.

Beispiel

I. Herstellung eines α-Hydroxy-ω-perfluoralkylalkanolpolyethers

170 g (ca. 0,37 Mol) Perfluoroctylethanol und 9,2 g Zinntetrachlorid werden in einem Druckreaktor unter Reinstickstoff auf 60 °C aufgeheizt und dazu über 3 h 163 g (ca. 2,8 Mol) Propylenoxid gegeben. Nach einer Nachreaktion mit der Dauer von 0,5 h wird abgekühlt. Die an einer Probe des Produktes ermittelte Epoxidzahl von 0,01 zeigt an, daß die Reaktion weitgehend beendet ist. Das Produkt wird mit 25 vol.-%igem Ammoniak neutralisiert, das Wasser bei 80 °C und 10 Torr abdestilliert und schließlich in Gegenwart eines Filterhilfsmittels filtriert.

Die maßanalytisch ermittelte Hydroxylzahl beträgt 63, was bei einer angenommenen Funktionalität von 1 einem Molekulargewicht von ca. 890 entspricht.

II. Herstellung eines α-Methacryloyl-ω-perfluoralkanolpolyethers

575 g (ca. 0,5 Mol) des α-Hydroxy-ω-perfluoralkanolpolyethers aus Beispiel 2 werden mit 0,4 g 2,6-Di-tert.Butylkresol in 1300 ml getrocknetem Chloroform gelöst. Zu dieser Lösung werden 60 g Triethylamin zugesetzt und der Ansatz auf 40°C erwärmt. Nun werden vorsichtig 53 g (ca. 0,5 Mol) Methacrylsäurechlorid zugetropft. Bei 50°C wird für 15 h nachgerührt. Anschließend wird zweimal mit 0,1 n HCl, viermal mit 5 %iger $NaHCO_3$-Lösung und mehrmals mit destilliertem Wasser bis zur Neutralität gewaschen. Die organische Phase wird mit $CaCl_2$ getrocknet und im Vakuum eingeengt. Man erhält 612 g einer leicht gelblichen, flüssigen Substanz mit dem theoretischen Molekulargewicht von 1219. Aus der NMR-spektroskopischen Untersuchung ergibt sich ein Umesterungsgrad von 99 %.

III. Herstellung eines weichbleibenden Unterfütterungsmaterials für Dentalprothesen

60 Gew.-Teile des α-Methacryloyl-ω-perfluoralkanolpolyethers werden mit 35 Gew.-Teilen 2,2,3,3,-Tetrafluoropropylmethacrylat und 5 Gew.-Teilen Perfluornonyl-1,2-diacrylat gemischt. Der Mischung werden 1,5 Gew.-% Dibenzoylperoxid zugesetzt.

Zu dieser Lösung werden durch intensives Mischen unter Vakuum im Labor-Planetenmischer 35 Gew.-Teile hydrophobe Kieselsäure zugegeben. Es entsteht eine klar-transparente Paste, die sowohl im Preß als auch im Injektionsverfahren als weiches Unterfütterungsmaterial für Dentalprothesen verwendet werden kann. Die Aushärtung erfolgt bei 70 bis 90°C im Wasserbad innerhalb von 1 bis 3 h.

Eine Aushärtung ist auch innerhalb weniger Minuten in handelsüblichen Mikrowellenherden möglich, wobei anschließend noch eine Stunde bei Raumtemperatur gelagert werden sollte.

Tabelle

| | Wasseraufnahme mg/cm² | Shore A Härte 37 °C |
|---|---|---|
| Erfindungsgemäß | 0,4 ± 0,1 | 28 - 33 |
| Handelsprodukt auf Silikonbasis | 1,70 ± 0,80 | 30 - 35 |
| Handelsprodukt auf Basis weichgemachter Methacrylate | 6,90 ± 1,20 | 47 - 50 |

## Patentansprüche

1. Verwendung von Gemischen von (Meth-)acrylsäureestern der allgemeinen Formel

$$R^1-(C_nF_{2n}-)-(CH_2-)_a-O-(C_mH_{2m}O-)_b-\underset{\underset{O}{\|}}{C}-\underset{\underset{R^2}{|}}{C}=CH_2$$

   wobei
   $R^1$ gleich oder verschieden und ein Wasserstoff- oder Fluorrest ist,
   $R^2$ gleich oder verschieden und ein Wasserstoff- oder Methylrest ist,
   a einen Wert von 0, 1, 2, 3 oder 4,
   b einen durchschnittlichen Wert von 2 bis 30,
   n einen durchschnittlichen Wert von 4 bis 12,
   m einen durchschnittlichen Wert von 3 bis 14 hat,
   mit der Maßgabe, daß im Molekülgemisch keine Oxyethylengruppen enthalten sind,
   als härtbare Makromonomere im Dentalbereich.

2. Verwendung von Gemischen von (Meth-)acrylsäureestern nach Anspruch 1 als Unterfütterungsmaterial für Zahnprothesen.

## Claims

1. Use of mixtures of (meth)acrylic acid esters of the general formula

$$R^1-(C_nF_{2n}-)-(CH_2-)_a-O-(C_mH_{2m}O-)_b-\underset{\underset{O}{\|}}{C}-\underset{\underset{R^2}{|}}{C}=CH_2$$

wherein
$R^1$ is identical or different and is a hydrogen or fluorine radical,
$R^2$ is identical or different and is a hydrogen or methyl radical,
a has a value of 0, 1, 2, 3 or 4,
b has an average value of 2 to 30,
n has an average value of 4 to 12 and
m has an average value of 3 to 14,
with the proviso that the mixture of molecules contains no oxyethylene groups,
as curable macromonomers in the dental sector.

2. Use of mixtures of (meth)acrylic acid esters according to Claim 1 as a lining material for dental prostheses.

## Revendications

1. Utilisation de composés d'esters de l'acide (méth)acrylique répondant à la formule générale

$$R^1-(C_nF_{2n}-)-(CH_2-)_a-O-(C_mH_{2m}O-)_b-\underset{\underset{O}{\|}}{C}-\underset{\underset{R^2}{|}}{C}=CH_2$$

où $R^1$ est identique ou différent et représente un reste d'hydrogène ou de fluor,
$R^2$ est identique ou différent et représente un reste d'hydrogène ou un reste méthyle,
a vaut 0, 1, 2, 3 ou 4,
b a une valeur moyenne de 2 à 30,
n a une valeur moyenne de 4 à 12,
m a une valeur moyenne de 3 à 14,
à la condition que le composé moléculaire ne contienne pas de groupes oxyéthylène,
en tant que macromonomères dans le domaine dentaire.

2. Utilisation de composés d'esters de l'acide (méth)acrylique selon la revendication, comme matériau de revêtement interne pour prothèses dentaires.